# EUROPÄISCHE PATENTSCHRIFT

(11) **EP 0 362 146 B1**
(45) Veröffentlichungstag und Bekanntmachung des Hinweises auf die Patenterteilung: **09.06.1993**
(21) Anmeldenummer: 89810728.9
(22) Anmeldetag: 26.09.1989
(51) Int. Cl.: A61M 29/02

(54) **Dilatationskatheter zur Aufweitung von Engstellen**
Dilatation catheter for stretching occlusions
Cathéter de dilatation pour élargir des occlusions

(30) Priorität: 27.09.1988 CH 3584/88
(43) Veröffentlichungstag der Anmeldung: 04.04.1990
(73) Patentinhaber: SCHNEIDER (EUROPE) AG, 8180 Bülach (CH)
(72) Erfinder: Ahmadi, Ramazan Ali, Prof. Dr., A-1180 Wien (AT); Bockenheimer, Stefan, Prof. Dr., D-6370 Oberursel/Ts. 4 (DE)
(74) Vertreter: Groner, Manfred

(56) Entgegenhaltungen:
- WO-A-86/05990
- GB-A- 2 187 390
- US-A- 4 406 656

## Beschreibung

Die Erfindung betrifft einen Dilatationskatheter nach dem Oberbegriff des unabhängigen Patentanspruchs 1.

Ein derartiger Dilatationskatheter ist aus der WO-A- 8 605 990 bekannt.

Dilatationskatheter dieser Art werden insbesondere zur Erweiterung von Engstellen im Arteriensystem benutzt. Zur Behandlung von Engstellen (Stenosen) in der Carotis konnten solche Dilatationskatheter aufgrund des hohen Risikos einer Embolie nicht verwendet werden. Bei der Behanldung von Engstellen in der Carotis musste deshalb bisher auf die vergleichsweise schonende Behandlung mit einem Katheter verzichtet werden.

Der Erfindung liegt die Aufgabe zugrunde, einen Dilatationskatheter der genannten Art zu schaffen, der insbesondere für die Aufweitung von Stenosen in der Carotis geeignet ist. Der Dilatationskatheter soll so beschaffen sein, dass die Behandlung bei möglichst kleinem Risiko dennoch für den Patienten schonend und sicher durchgeführt werden kann.

Die Aufgabe wird durch die Erfindung gemäss Anspruch 1 gelöst. Der Dilatationskatheter kann in bekannter Weise mit Hilfe eines Führungsdrahtes in das zu behandelnde Gefäss eingesetzt werden. Mit dem Dichtungsballon lässt sich in Strömungsrichtung des Blutes gesehen nach der Engstelle das Gefäss so abdichten, dass keine bei der Behandlung der Engstelle möglicherweise sich lösende Partikel weggeschwemmt werden können. Damit wird die Gefahr einer Embolie vermieden. Nach dem Abdichten des Gefässes kann die Engstelle mit dem Dilatationsballon in an sich bekannter Weise mechanisch ausgeweitet werden. Hierbei sich lösende Teilchen bleiben im Bereich der Engstelle und können nach der Behandlung der Engstelle durch die zwischen den beiden Ballons angeordnete Absaug bzw. Einspülvorrichtung vollständig entfernt oder weggespült werden. Vorteilhafte Weiterbildungen ergeben sich aus den abhängigen Ansprüchen.

Ein Ausführungsbeispiel der Erfindung wird nachfolgend anhand der Zeichnungen näher erläutert. Es zeigen:
- Fig. 1a: in vergrössertem Massstab eine Seitenansicht des vorderen Teils eines erfindungsgemässen Dilatationskatheters,
- Fig. 1b: eine Seitenansicht des hinteren Teils des Dilatationskatheters,
- Fig. 2: einen Querschnitt durch den Dilatationskatheter entlang der Linie II-II in Fig. 1, und
- Fig. 3a bis 3d: in schematischer Darstellung einen Abschnitt der Carotis mit eingesetztem Kathetervorderteil in verschiedenen Phasen während der Behandlung einer Engstelle.

Der Dilatationskatheter weist einen flexiblen Trägerschlauch 6 mit einem durchgehenden zentralen Kanal 11 zur Aufnahme eines ebenfalls flexiblen Führungsdrahtes 7 auf. Mit Hilfe des Führungsdrahtes 7 kann das vordere Ende des Dilatationskatheter im zu behandelnden Gefäss 19 zu einer Längsstelle 20 vorgeschoben werden. Zur Aufweitung der Engstelle 20 ist ein bei der Einführung des Katheters gefalteter Dilatationsballon 2 am Trägerschlauch 6 angebracht, der mit einer hier nicht gezeigten, an einem Nippel 16 anzuschliessenden Druck-Saugpumpe bis zu einem bestimmten Umfang regulierbar aufgeblasen werden kann. Die Druck-Saugpumpe wird an einem Nippel 16 angeschlossen und ist über einen Druckkanal 8, der mit Oeffnungen 3 im Innenraum des Dilatationskatheters endet, mit diesem Innenraum verbunden. Zur röntgenographischen Lokalisierung des Dilatationsballons 2 sind am Trägerschlauch 6 zwei metallische Ringe angebracht. Wesentlich ist, dass der Dilatationsballon 2 ein bei den hier üblichen Drucken sehr geringe Elastizität aufweist und nicht über den genannten bestimmten Umfang ausgedehnt werden kann.

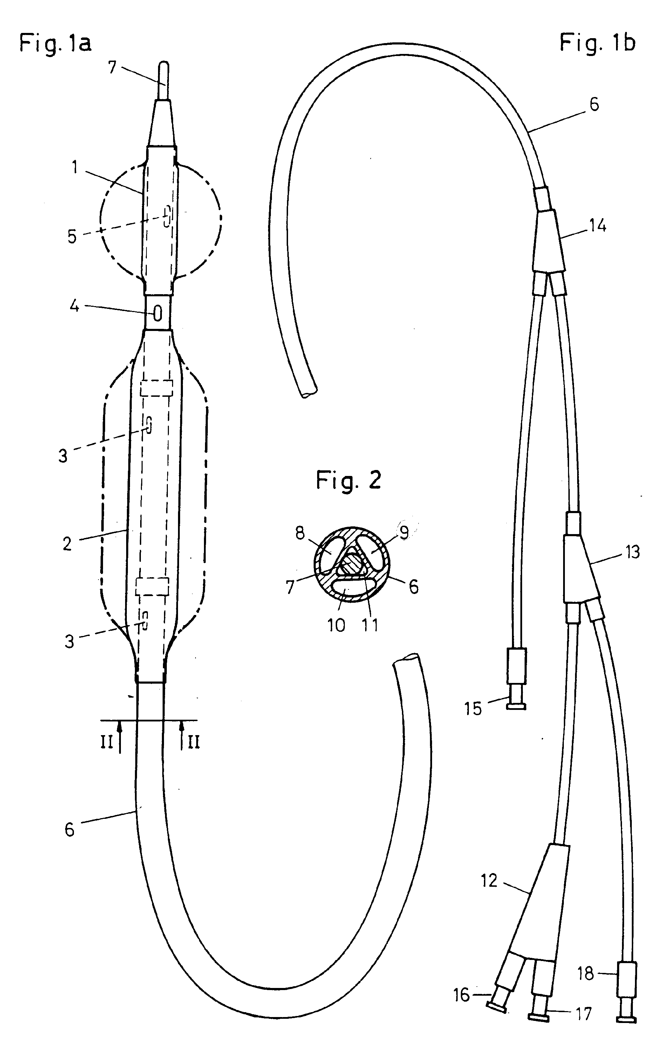

Vor dem Dilatationsballon 2 und im Abstand zu diesen ist ein Dichtungsballon 1 angebracht, der in Ruhestellung eng am Trägerschlauch 6 anliegt. Dieser Ballon ist etwa 1 bis 2 cm lang und an seinen beiden Enden dicht mit dem Trägerschlauch 6 verbunden beispielsweise mit dem Trägerschlauch verschweisst. Im Gegensatz zum Dilatationsballon 2 ist nun dieser Dichtungsballon 1 gummielastisch und kann unter Dehnung der Wandung ausgedehnt werden. Vorzugsweise ist der Dichtungsballon 1 aus Latex hergestellt. Mit einer hier nicht gezeigten Druck-Saugpumpe kann der Dichtungsballon 1 regulierbar in die in Fig. 1a strichpunktiert eingezeichnete kugelförmige Form aufgeblasen werden. Diese Druck-Saugpumpe wird am Nippel 18 angeschlossen und ist über einen Druckkanal 9 mit dem Innenraum des Dichtungsballon 1 verbunden. Der Kanal 9 führt über eine Oeffung 5 im Trägerschlauch 6 in diesen Innenraum.

Zwischen dem Dichtungsballon 1 und dem Dilatationsballon 2 weist der Trägerschlauch 6 eine Absaug- Einspülöffnung 4 auf, die direkt in das Gefäss 19 mündet und über einen Kanal 10 zu einem Nippel 15 führt, der an eine hier ebenfalls nicht gezeigtes Absaug- Einspülgerät anzuschliessen ist. Durch diese Absaug-Einspülöffnung 4 können während und nach der Behandlung Teilchen abgesaugt oder weggespült werden, die während der Aufweitung der Engstelle von dieser abgelöst worden sind.

Der Trägerschlauch 6 ist von den Oeffnungen 3 bis zu einer Abzweigung 14 vierlumig, von dieser Abzweigstelle 14 bis zur Abzweigstelle 13 dreilumig und auf der hintersten Strecke bis zum Abzweigstück 12 zweilumig. Die Nippel 15 und 18 sind über einlumige Schlauchstücke an den entsprechenden Abzweigstellen angeschlossen. Die Kanäle zu den Oeffnungen 3, 4 bzw. 5 sind getrennt, so dass die beiden Ballone 1 bzw. 2 unabhängig voneinander aufgeblasen werden können und unabhängig vom Zustand dieser Ballone durch die Oeffnung 4 abgesaugt oder eingespült werden kann.

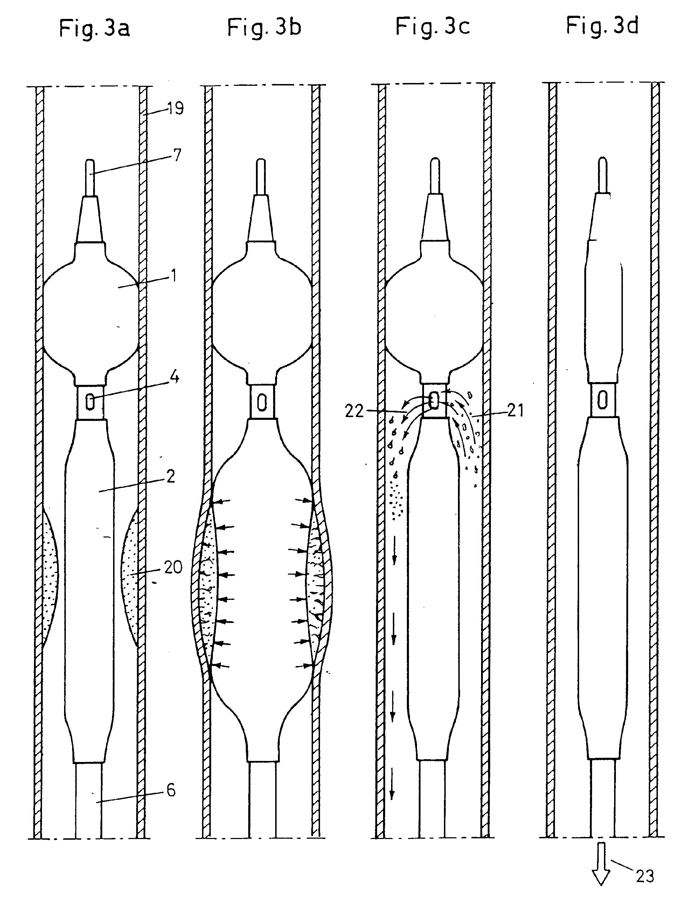

Anhand der Fig. 3a bis 3d wird die Anwendung des Dilatationskatheters näher erläutert.

Das vordere Ende des Dilatationskatheters wird mit Hilfe des Führungsdrahtes 7 im Gefäss 19 bis zur Engstelle 20 vorgeschoben. Der Dilatationsballon 2 ist hierbei gefaltet und der Dichtungsballon 1 liegt entspannt am Trägerschlauch 6 an. Ist der Dilatationsballon 2 positioniert, so wird der Dichtungsballon 1 soweit aufgeblasen, dass er innenseitig am Gefäss 19 anliegt und dieses soweit abdichtet, dass an dieser Stelle keine Körperflüssigkeit mehr hindurchfliessen kann. Dieser Zustand ist in Fig. 3a dargestellt, wo auch klar ersichtlich ist, dass sich der weiche und gummielastische Dichtungsballon 1 ohne wesentliche Dehnung des Gefässes 19 an diesem dichtend angelegt hat.

Als nächster Schritt wird der Dilatationsballon 2 aufgeblasen wobei er mit zunehmendem Umfang die Engstelle 20, die in der Regel aus schwammigem, kalk- und fettreichem Gewebe besteht, aufweitet. Durch diese Aufweitung wird das die Engstelle bildende Gewebe teilweise in die Gefässwand hineingedrückt.

Nun wird der Dilatationsballon 2 wie in Fig. 3c gezeigt in die geschrumpfte Form gebracht und gleichzeitig oder kurz vorher durch die Absaug-Einspülöffnung 4 Körperflüssigkeit und damit möglicherweise losgelöstes Engstellenmaterial aus dem Gefäss 19 in Richtung der Pfeile 21 und/oder durch eine Einspülung in Richtung der Pfeile 22 (Fig. 3c) entfernt. Schliesslich wird der Dichtungsballon 1 wieder in die wie Fig. 3d gezeigte entspannte Form gebracht und der Katheter so wie der Führungsdraht 7 aus dem Gefäss 19 entfernt. Einzelne genannte Behandlungsschritte können selbstverständlich auch mehrmals wiederholt werden.

Der erfindungsgemässe Behandlungskatheter eignet sich insbesondere für die Behandlung von Engstellen in der Carotis.

## Patentansprüche

1. Dilatationskatheter zur Aufweitung von Engstellen (20) in einem Blutgefäss (19), mit einem an einem Trägerschlauch (6) angeordneten Dilatationsballon (2), der von aussen mittels einer am Trägerschlauch (6) anzuschliessenden Druck-Saugpumpe bis zu einem bestimmten Umfang ausdehnbar ist, wobei im Abstand zum Dilatationsballon (2) ein Dichtungsballon (1) angeordnet ist, dessen Wandung von aussen über einen Druckkanal (9) des Trägerschlauchs (6) elastisch ausdehnbar ist, um das Gefäss (19) im Bereich der Engstelle (20) abzudichten, und wobei der Trägerschlauch (6) zwischen dem Dilatationsballon (2) und dem Dichtungsballon (1) eine Oeffnung (4) zum Einleiten von Flüssigkeit aufweist, dadurch gekennzeichnet, dass der Druckkanal (9) einzig mit dem distal des Dilatationsballons (2) angeordneten Dichtungsballon (1) verbunden ist, derart, dass mittels dieses Dichtungsballons (1) das Gefäss (19) lediglich an einer Stelle distal des Dilatationsballons (2) abdichtbar ist und das Gefäss (19) bei entspanntem Dilatationsballon (2) proximal der Oeffnung (4) frei ist, und daß diese Oeffnung (4) eine Absaug- bzw. Einspülöffnung ist, die hinreichend gross ist, damit durch diese losgelöstes Engstellenmaterial mittels eines am proximalen Ende des Trägerschlauches (6) angeschlossenen Absaug- und Einspülgerätes in den Trägerschlauch (6) absaugbar ist oder Flüssigkeit durch diese Oeffnung (4) bei gefaltetem Dilatationsballon (2) und gedehntem Dichtungsballon (1) in das proximal dieses Dichtungsballons (1) freie Gefäss einspülbar ist, um losgelöstes Engstellenmaterial entlang des Trägerschlauches von der Engstelle (20) wegzuführen.

2. Dilatationskatheter nach Anspruch 1, dadurch gekennzeichnet, dass der Dichtungsballon aus Latex hergestellt ist.

3. Dilatationskatheter nach Anspruch 1 oder 2, dadurch gekennzeichnet, dass der Dichtungsballon (1) im Ruhezustand am Trägerschlauch (6) anliegt.

4. Dilatationskatheter nach einem der Ansprüche 1 bis 3, dadurch gekennzeichnet, dass der frei aufgeblasene Dichtungsballon (1) kugelförmig ist.

5. Dilatationskatheter nach einem der Ansprüche 1 bis 4, dadurch gekennzeichnet, dass der Dichtungsballon (1) radial über den genannten bestimmten Umfang des Dilatationskatheters (2) dehnbar ist.

6. Dilatationskatheter nach einem der Ansprüche 1 bis 5, dadurch gekennzeichnet, dass durch die Absaug- bzw. Einspülöffnung (4) bei aufgeblasenem Dichtungsballon (1) und gefaltetem Dilatationsballon (2) abgesaugt bzw. eingespült werden kann.

7. Dilatationskatheter nach Anspruch 6, dadurch gekennzeichnet, dass der Trägerschlauch (6) vierlumig ausgebildet ist, und ein zentraler Kanal (11) für einen Führungsdraht (7) vorgesehen ist.

## Claims

1. A dilatation catheter for widening occlusions (20) in a blood vessel (19), by means of a dilating balloon (2) arranged on a carrier hose (6), said dilating balloon being extendable up to a specific size, from outside, by means of a pressure-suction pump to be connected to the carrier hose (6), a sealing balloon (1) being arranged at a spacing from the dilating balloon (2), the walls of which sealing balloon being resiliently extendable from outside via a pressure channel (9) of the carrier hose (6) in order to seal the vessel (19) in the region of the occlusions (20), and the carrier hose (6) having an opening (4) between the dilating balloon (2) and the sealing balloon (1) for introducing liquid, characterised in that the pressure channel (9) is only connected to the sealing balloon (1) arranged distally of the dilating balloon (2), such that the vessel (19) may only be sealed, by means of this sealing balloon (1), at a position distally of the dilating balloon (2) and the vessel (19) is clear proximal to the opening (4) when the dilating balloon (2) is deflated, and that this opening (4) is an extraction or supply opening which is sufficiently large for material which has become detached from the occlusion to be suctioned off into the carrier hose, by means of an extraction and supply unit connected to the proximal end of the carrier hose (6), or, when the dilating balloon is deflated and the sealing balloon is extended, for liquid to be introduced, via said opening (4), into the vessel, cleared proximally to said sealing balloon (1), in order to carry the detached material away from the occlusion (20) via the carrier hose.

2. A dilatation catheter according to claim 1, characterised in that the sealing balloon is made of latex.

3. A dilatation catheter according to either claim 1 or 2, characterised in that the sealing balloon (1) bears against the carrier hose (6) in the rest position.

4. A dilatation catheter according to any one of claims 1 to 3, characterised in that when freely inflated, the sealing balloon (1) is spherical.

5. A dilatation catheter according to any one of claims 1 to 4, characterised in that the sealing balloon (1) can be extended radially beyond the said specific size of the dilatation catheter (2).

6. A dilatation catheter according to any one of claims 1 to 5, characterised in that the extraction or supply via the extraction or supply opening (4) can be carried out when the sealing balloon is inflated and the dilating balloon is deflated.

7. A dilatation catheter according to claim 6, characterised in that the carrier hose (6) is constructed with four openings and that a central channel (11) is provided for a guide wire (7).

## Revendications

1. Cathéter de dilatation pour l'élargissement de rétrécissements (20) dans un vaisseau sanguin (19), avec un ballon de dilatation (2), disposé sur un flexible de support (6), qui peut être dilaté de l'extérieur, à l'aide d'une pompe de refoulement-aspiration à raccorder au flexible de support (6), jusqu'à un volume déterminé, à une distance du ballon de dilatation (2) étant disposé un ballon d'obturation (1) dont la paroi peut être dilatée élastiquement de l'extérieur, par un canal à pression (9) du flexible de support (6), pour obturer le vaisseau (19) à l'endroit du rétrécissement (20) et le flexible de support (6) présentant, entre le ballon de dilatation (2) et le ballon d'obturation (1), un orifice (4) pour l'introduction de liquide, caractérisé en ce que le canal à pression (9) est raccordé uniquement au ballon d'obturation (1) disposé distalement par rapport au ballon de dilatation (2), de telle manière que, à l'aide de ce ballon d'obturation (1), le vaisseau (19) ne peut être obturé qu'à un seul endroit distal par rapport au ballon de dilatation (2) et que le vaisseau (19), lorsque le ballon de dilatation (2) est détendu, est libre du côté proximal par rapport à l'orifice (4), et en ce que cet orifice (4) est un orifice d'aspiration, respectivement de refoulement qui est suffisamment grand pour que par celui-ci peut être aspirée dans le flexible de support (6), à l'aide d'un appareil d'aspiration et de refoulement raccordé à l'extrémité proximale du flexible de support (6), de la matière du rétrécissement détachée ou pour que du liquide peut, par cet orifice (4), lorsque le ballon de dilatation (2) est plié et que le ballon d'obturation (1) est dilaté, être refoulé dans le vaisseau libre du côté proximal par rapport à ce ballon d'obturation (1), afin d'évacuer du rétrécissement (20), le long du flexible de support, la matière du rétrécissement détachée.

2. Cathéter de dilatation suivant la revendication 1, caractérisé en ce que le ballon d'obturation est réalisé en latex.

3. Cathéter de dilatation suivant la revendication 1 ou 2, caractérisé en ce que le ballon d'obturation (1) s'appuie, à l'état de repos, sur le flexible de support (6).

4. Cathéter de dilatation suivant l'une des revendications 1 à 3, caractérisé en ce que le ballon d'obturation (1) librement gonflé est de forme sphérique.

5. Cathéter de dilatation suivant l'une des revendications 1 à 4, caractérisé en ce que le ballon d'obturation (1) peut être dilaté radialement suivant ledit volume déterminé du cathéter de dilatation (2).

6. Cathéter de dilatation suivant l'une des revendications 1 à 5, caractérisé en ce que, à travers l'orifice d'aspiration, respectivement de refoulement (4), il peut être aspiré ou refoulé lorsque le ballon d'obturation (1) est gonflé et le ballon de dilatation (2) est plié.

7. Cathéter de dilatation suivant la revendication 6, caractérisé en ce que le flexible de support (6) est réalisé avec quatre ouvertures et qu'il est prévu un canal central (11) pour un fil de guidage (7).
